Europäisches Patentamt

European Patent Office

Office européen des brevets

(19)

(11) Publication number: **0 224 947 B1**

# EUROPEAN PATENT SPECIFICATION

(12)

(45) Date of publication of patent specification: **31.07.91**      (51) Int. Cl.⁵: **B01J 37/02**

(21) Application number: **86201917.1**

(22) Date of filing: **31.10.86**

(54) A process for loading a shaped carrier material with a catalytically active material or with a precursor of a catalytically active material, and a shaped catalyst produced by said process.

(30) Priority: **01.11.85 NL 8502992**

(43) Date of publication of application:
**10.06.87 Bulletin 87/24**

(45) Publication of the grant of the patent:
**31.07.91 Bulletin 91/31**

(84) Designated Contracting States:
**BE DE ES FR GB IT NL SE**

(56) References cited:
**DE-B- 1 072 595**
**FR-A- 2 359 642**
**GB-A- 2 131 714**
**US-A- 3 843 745**

(73) Proprietor: **THE DOW CHEMICAL COMPANY**
**2030 Dow Center Abbott Road**
**Midland, MI 48640(US)**

(72) Inventor: **Wigman, Johannes Marinus**
**Schorrenkruidlaan 38**
**NL-4553 BX Philippine(NL)**
Inventor: **Bongaarts, Jacobus Elisabeth**
**Admiraalstraat 196**
**B-9120 Destelbergen(BE)**
Inventor: **Geus, John Wilhelm**
**Gezichtslaan 100**
**NL-3723 GJ Bilthoven(NL)**
Inventor: **Meima, Garmt Ricardo**
**Aalberselaan 4c**
**NL-3818 XM Amersfoort(NL)**

(74) Representative: **Smulders, Theodorus A.H.J.,**
**Ir. et al**
**Vereenigde Octrooibureaux Nieuwe Parklaan**
**107**
**NL-2587 BP 's-Gravenhage(NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for preparing a carrier material for loading with a catalytically active material or with a precursor of a catalytically active material.

In solid catalysts, the catalytic activity is generally bound to the surface of the solid. For this reason the area of the active surface and its accessibility to reactants are co-determinative of the activity and selectivity of the catalyst. Activity, as used in this context, means the conversion of the reactants per unit volume of the catalyst and selectivity means the degree in which a desired reaction is accelerated relative to the acceleration of undesirable reactions. According to this definition of the activity, the area of the catalytically active surface per unit volume of the catalyst is determinative.

A high activity, therefore, requires a large active area per unit volume, which can only be achieved with small particles of the active component. The need of using catalytically active components in finely-divided form has a number of major drawbacks:

- solid catalysts must generally have a high mechanical strength; it is difficult, however, to process finely-divided material to mechanically strong moldings while retaining a high porosity, because the pore structure thus obtained is generally unfavorable; in fact, often narrow pores with a (too) large effective pore length ('tortuousness'):
  besides, in the case of many catalytically active elements and compounds, processing to mechanically strong moldings is not possible at all.
- Many catalytic reactions are carried out at temperatures and in gas atmospheres whereby small particles of the active component are rapidly sintered to form much larger particles; the concomitant decrease in active area leads to a rapid loss in activity.
- As the pores in a system of very small particles are mostly narrow, the transport of reactants and reaction products in the catalyst is difficult. On the one hand, this leads to a less high activity of the catalyst, while its selectivity is also adversely affected.

To overcome the problems outlined above, so-called carrier materials are rather generally used in heterogeneous catalysis. A carrier material can be satisfactorily processed to thermostable, mechanically strong bodies of the desired dimensions, while the pore structure of the carrier can be effectively adjusted. The catalytically active material, or a precursor thereof, is applied to the carrier surface, mostly in extremely finely-divided form. Generally speaking, the surface of the carrier material is not catalytically active. Although, therefore, the carrier greatly dilutes the catalytically active material, the fact that the catalytically active component is no longer sintered at high temperatures causes the thermostable catalytically active area to be considerably higher than without the use of a carrier.

To promote as efficient a utilization of the carrier material as possible, the active component must be applied to the carrier surface in finely-divided form and as homogeneously as possible. In principle, it is possible to load small particles of the desired carrier material relatively simply with (a precursor of) the active component in such a manner that it is uniformly distributed over the carrier surface as small particles. Precursor as used in this context means the element or compound which later is converted into the catalytically active component, for example, by a thermal treatment or some other reaction.

In the preparation of solid catalysts on a technical scale, the application of the active component to the carrier is in many cases problematic. In fact, mostly, the active component cannot be applied to the carrier. Thus in the case of many metals, it is impossible for these two to be directly applied to a carrier in finely-divided form. In such cases, for example, a hydrated oxide of the metal - the catalytic precursor - is applied to a carrier in finely-divided form, whereafter during a separate thermal pretreatment the metal oxide is reduced to the metal.

When small particles of the carrier material are used the pores present in it are short, owing to which the migration of the precursor of the active component into the carrier particle can readily proceed. Small particles, however, cannot be used in some much-used catalytic reactors, namely, those in which the catalyst is present in a solid bed. When small particles are used, the pressure drop across the catalyst bed becomes intolerably high. Also, 'channelling' often occurs, which is the phenomenon that the reactants flow through a limited part of the cross-section of the reactor only, namely, at the places where the particles are in motion.

A necessary condition which technical catalysts have to satisfy is the possession of a high mechanical strength. During the filling of the reactor, mostly substantial forces are exerted on the catalyst body, while during the starting-up and stopping of the reactor, these bodies are often subjected to large differences in temperature. Breakage of catalyst bodies in the reactor is highly undesirable. This leads to poor distribution of the reactants over the cross-section of the catalyst bed or over a number of parallel-connected reactor tubes.

In some, but not all, cases, it is possible to load a powdered carrier first with (a precursor of ) the active component - for example, as described above - and subsequently to process the composite to larger moldings having the necessary mechanical strength.

Many catalyst systems which, by themselves, are attractive, have never been applied on a technical scale, because it was impossible for them to be processed to bodies having the required mechanical strength. For this reason there has been a need for a long time of a technically feasible method of loading shaped carrier bodies with (a precursor of) the active component. In fact, using this, first carrier bodies of the desired sizes and mechanical strength can be made - which commonly can be satisfactorily effected - whereafter subsequently these bodies are loaded with the (precursor of the) active component.

The methods used according to the state of the art for applying (precursors of) the active component(s) to carrier bodies do not usually lead to the desired uniform distribution of the active component(s). The procedure most commonly used is the impregnation of the carrier bodies with a solution of a precursor of the active material, the solvent of which is removed by drying. often, however, it is observed that the precursor of the active material is only deposited on the outer surface of the carrier bodies or at the pore mouths.

Some authors believe that viscous flow owing to capillary forces is the driving force behind the migration to the outside of the carrier body of the elements of the solution remaining behind during drying (N. Kotter and L. Riekert in ?preparation of catalysts II', B. Delmon, P. Grange, P. Jacobs and G. Poncelet, eds., pp. 51-63, Elsevier, Amsterdam, 1979.

These authors have therefore proposed to use a viscous solution of (a precursor of) the active material in the impregnation. As the authors state, the use of a viscous solution does indeed lead to a somewhat more homogeneous distribution of the active material over the carrier. One disadvantage of impregnating with a viscous solution is, however, that the viscous solution cannot well penetrate into long, relatively narrow pores, as generally occur in shaped catalyst bodies.

Other authors believe that the gas which remains behind in the pores of the carrier body during the impregnation forces the elements of the solution outside (S.Y. Lee and R. Aris in 'preparation of Catalysts III', P. Poncelet, P. Grange and P.A. Jacobs, eds. pp. 35-45, Elsevier, Amsterdam, 1983). As can be inferred from the detailed publication by Lee and Aris, many factors which are difficult to control play a role in the impregnation and drying of catalyst pellets. The result is that the distribution of the active component after impregnation and drying is often far from uniform.

As observed above, this is generally unfavorable for the activity of the catalyst. In some cases, where poisoning of the active component occurs, the active material is deliberately distributed non-uniformly over the carrier body, but mostly this is unattractive.

In order to render the distribution more homogeneous, the above-cited Kotter and Riekert have proposed to increase the viscosity of the solution used for impregnation by adding hydroxyethyl cellulose to it. It is true that this results in a homogeneous distribution of the precursor, but a very important drawback of this method is that a viscous solution of a precursor can hardly, if at all, penetrate the narrow pores of a carrier body, so that ultimately no homogeneous distribution is obtained during the impregnation. In the preparation of supported catalysts of which the carrier has a small surface area (BET), an additional problem is, that an even and dense distribution of catalytically active material or precursor therefore, is very difficult to obtain. In the prior art carriers commonly used have a very large surface area, for example well above 25 $m^2/g$. With a high surface area, the capillary activity of the carrier takes care of getting a sufficient amount of catalytically active material in the pores.

With a carrier having a low surface area this phenomenon does not occur, with the result that the distribution will become uneven if no specific methods are used.

According to another known method, the so-called deposition-precipitation, an amount of solution of the active component is added just sufficient to fill the pores of the carrier body. This method is also sometimes referred to as an embodiment of dry impregnation or 'incipient wetness' impregnation. In addition to the active component, the solution contains urea, ammonium cyanate or another compound which upon hydrolysis increases the pH. After the bodies have been impregnated at such a low temperature that there is no appreciable hydrolysis yet, the temperature is increased. An active component or a precursor of the active component can then deposit on the surface of the carrier, provided there is a sufficient interaction between the nuclei of the precipitating precursor and the carrier surface. In some cases, however, this process cannot be used. On the one hand these are cases where there is no good interaction with the carrier; and on the other hand there are a number of important active materials which cannot be precipitated by increasing the pH of the liquid.

There is another condition which should be satisfied in developing a universal method of applying a precursor of an active material to carrier bodies, namely, that the accessible area and the pore distribution of the resulting laden carrier should not differ from that of the non-laden carrier material. Thus, in particular to obtain a good selectivity, the pore structure of the ultimate catalyst must be capable of being effectively adjusted. Accordingly, the application of the active component must not appreciably affect the suitable pore structure of the carrier. This latter is especially to be feared if the active component is deposited on the carrier in the form of small clustered particles. In that case the often narrow pores between the small particles of the active component lead to an additional porosity which is mostly unfavorable.

FR-A 2,359,642 discloses a process for preparing a supported catalyst wherein shaped carrier materials having a surface area below 10m$^2$/g are impregnated with a silver compound or a silver complex. Subsequently the impregnated material is dried and heated to decompose the silver compound or complex.

DE-A 1,072,595 describes the preparation of supported catalyst by impregnation of porous carriers with a solution of a metal complex.

The problem underlying the present invention, therefore, was to provide a generally suitable process for applying an active component to shaped carrier materials, which carrier materials are characterized by a low surface area (below 20 m$^2$/g), without appreciably changing the accessible area and the pore structure, and which process gives an even and dense distribution of the active component.

The invention is characterized by impregnating the carrier which has a surface area of less than 20 m$^2$/g with a solution of a complex of tin or iron and citric acid, formic acid, lactic acid or oxalic acid in a solvent, the viscosity of which solution is not decreased upon heating and/or evaporation of the solvent, subsequently evaporating the solvent and decomposing the complex by heating. It has surprisingly been found that by using such a process the compound of the active component remains homogeneously distributed in the pores of the carrier. The term 'catalytically active material' or 'active material' as used herein is a generic term which includes catalytic components such as zero valent metals, catalytic compounds, such as lower valent metal ions and precursors for catalytic components.

According to one embodiment of the process according to the invention, a solution of said complex is used, the viscosity of which increases upon heating and evaporation of the solvent.

According to another embodiment, a solution of said complex is used, in which the crystallization of the solid complex does not proceed, or at a low rate, upon heating and evaporation of the solvent.

According to a highly suitable embodiment of the process according to the invention, the bodies of the carrier, material are impregnated with so much of the solution of said complex as is needed for just filling the pore volume of the carrier.

According to a preferred process according to the invention, the carrier material is evacuated prior to the impregnation with the solution of a complex of the active component, if necessary at elevated temperature. It is noted that the use of, for example, citric acid in the impregnation of bodies of carriers is known per se. Suitable combinations of components in the solution, i.e. solvent active component and chelating or viscosity enhancing agent or mixture of agents can be determined by simple experiments monitoring the viscosity of the solution during evaporation.

In this connection reference can be made to E.R. Becker and T.A. Nuttall in 'Preparation of Catalysts II', B. Delmon, P. Grange and P. Jacobs and G. Poncelet, eds., pp. 159-170, Elsevier, Amsterdam, 1979 and G.H. van den Berg and H.Th. Rijnten, ibidem, pp. 265-277. In that case, however, the acid is added to produce a non-homogeneous distribution of, especially, noble metals over aluminum oxide bodies. The acid exhibits competitive adsorption with a negatively-charged noble metal complex, such as, for example, hexachloro platinic acid, as a result of which the platinum is only deposited in the middle of the carrier body. In this procedure, generally speaking, the impregnation period is kept relatively short, and the thermal decomposition of a complex with citric acid does not take place.

The carrier materials to be used in the process according to the invention are such as are known in heterogeneous catalysis. Examples of suitable carrier materials are oxidic compounds, such as silicon dioxide, aluminum oxide, silicon oxide/aluminum oxide, magnesium oxide, zirconium oxide and titanium dioxide. Examples of other carrier materials are carbon and carriers used less often, such as silicon nitrides or carbides. According to a preferred embodiment the surface area is less than 15 m$^2$/g. In the most preferred embodiment the surface area is less than 10 m$^2$/g.

The solution of a metal complex whose viscosity does not decrease upon heating, as used in accordance with this invention, can be obtained in several ways. First it is possible to use a solution , which is in such a form that, upon evaporation of the solvent, the viscosity is increased. This is basically the case with compounds that do not properly crystallize. Another possibility is the use of a solution of a compound whose viscosity even increases upon heating. A third possibility consitsts in adding a substance which has such an effect on the solution of the active component that a higher viscosity is obtained upon heating.

Generally speaking, the use of a solution of the metal complex from which no, or substantially no, crystallization (of a compound) of the active component occurs is preferred.

In accordance with a suitable embodiment of the process according to invention, this can be achieved by adding to the solution of the metal complex sugar, glucose, fructose or similar compounds which are readily soluble and poorly crystallizable. The concentration of the poorly crystallizing compound is selected so that the viscosity of the solution with which the carrier is impregnated is not much higher than that of the solution which only contains the (precursor of the) active component.

In order that the (precursor of the) metal may be deposited over the (interior) surface of the bodies of the carrier material as uniformly as possible, it is of importance that, as the solution is heated its viscosity is increased so that no, or substantially no, migration of the precursor to external surface of the body of the carrier material takes place.

After the removal of the solvent and thermal decomposition of the complex, the material deposited on the carrier mostly still contains carbonaceous products. These can be removed, for example, by oxidation at elevated temperature. Owing to the uniform distribution of the catalytically active precursor over the carrier surface, the active material is not sintered during the oxidation at elevated temperature. When the active component must be used in reduced form, the active material can be reduced. Reduction can be effected with gaseous reducing agents, such as hydrogen or carbon monoxide at elevated tempertures. By adjusting the hydrogen/water or the carbon monoxide/carbon dioxide ratio, the degree of reduction can be controlled, for example, to a lower oxide or to the corresponding metal. The reduction may also be carried out at lower temperature with a dissolved reducing agent. Examples of dissolved reducing agents are hydrazine, hydroxylamine, litium boron hydride, or sodium aluminum hydride. Weaker reducing agents that can be used in the dissolved state are formaldehyde, methanol or glucose.

A major advantage of the process according to the invention is that it makes it possible for an active material with a large specific area to be applied to a carrier having wide pores, and hence a surface that is well accessible, without conglomerates of small particles of the active material leading to the presence of narrow pores in the catalyst system. Especially in the case of catalysts for selective reactions, such as the oxidation of ethylene to ethylene oxide, or of methanol to formaldehyde, it is important that the catalysts should not contain narrow pores. The relatively long residence time of the reaction product in the narrow pores leads to oxidation and the formation of undesirable products.

The process according to this invention is excellently suitable for applying silver to bodies of alpha-aluminum oxide. In this case, tin (II) or tin (IV) is applied to the carrier via a citrate complex using the process of this invention. The process according to the invention brings about that the tin oxide which results after drying of the impregnated carrier, decomposition of the complex and oxidation of the carbonaceous products is deposited as a continuous layer on the aluminum oxide surface. The tin oxide can be reduced, for example, with hydrogen or with formaldehyde to form tin(II)oxide. If, subsequently, it is reacted with dissolved silver ions, the silver is deposited upon the carrier surface uniformly and in finely-divided form. As tin oxide leads to excellent adherence of the silver to the carrier, the resulting silver catalyst is extremely thermostable.

Of great importance is that the process is excellently suitable for large-scale application. Large quantities of carrier bodies may very well be impregnated with a solution of an active precursor by the 'incipient wetness' method. Extraordinarily good results are achieved when the bodies of the carrier material are previously evacuated. Preferably such evacuation is effected at elevated temperature. Filling the interior pore system of the bodies of the carrier material then proceeds exptremely readily. Drying and heating the impregnated body, too, can be effected on a large scale without any objection.

The process according to the invention is also particularly suitable for applying a carrier material to a specific structure, such as, for example, a 'monolithic' or 'honeycomb' structure. These non-porous structures are used on account of their low flow resistance. A thermostable finely-divided material must be applied to the surface of the structure; to this thermostable material, a catalytically active component is applied. According to the state of the art, this is effected by immersing the structure in a suspension of, for example, aluminum oxide or silicon dioxide in finely to extremely finely divided form ('dip coating'). Subsequently, the structure is kept at an elevated temperature to fix the small particles on the surface of the structure. In these cases, however, the density of the small particles that can be applied per dip is rather poor. Often, the structure must be immersed many times to produce a fair surface. In the process according to the present invention, an aliminum oxalate solution is, for example, applied to the structure. When the oxalate solution is previously heated, its viscosity can be adjusted so that a sufficient amount of the solution can be applied to the structure in one step.

The invention is illustrated in and by the following examples.

## Example 1

0.85 g Sn(II)oxalate (Fluka A.G., purum) was introduced together with 0.79 g citric acid (Merck pa.) in about 15 ml deionized water. The pH was increased with concentrated ammonia (25% by weight) to a value of 5.8, at which the solution was clear. In this solution the bivalent tin is present as the citrate complex. If no complexing agent is present, the Sn(II) would hydrolyse at this pH. The solution was made up with deionized water to a final volume of 21.5 ml.

50.0 g alpha-aluminum oxide pellets (Norton SA 5525 HPC] were then impregnated with the above solution by the incipient wetness procedure. These pellets are cylindrical with a diameter of about 5 mm and a length of about 7 mm and have a BET area of 0.27 $m^2$/g. The pellets thus treated were dried for about 30 minutes at 120°C and, on the basis of Sn(O) contain 0.97% by weight of Sn. Subsequently, the BET area was measured after 20 hours' calcination as a function of the calcination temperature. After drying at 120°C, no increase in area was to be measured relative to that of the pure carrier material. This indicates that there has been no decomposition in the complex and that, accordingly, no $SnO_2$ particles have formed. After the calcination at 500°C, an area of 0.86, approximately 0.02$m^2$/g was measured; at 750°C 1.01, approximately 0.04 $m^2$/g; at 850°C 0.68, approximately 0.01 $m^2$/g: at 1000°C and at 1250°C, no increase in area relative to that of the pure carrier material was measured.

The above results indicate that the $SnO_2$ is sintered as a function of the temperature after first being deposited on the $Al_2O_3$ surface as extremely small particles at low temperature. This was confirmed with mercury porosimetry. Electron microscopy confirmed the above picture; in addition, it was shown by means of elemental analysis throughout the entire pellet. Sometimes a somewhat higher load was observed on the outside of the pellet. analysis in the scanning microscope that the $SnO_2$ distribution is homogeneous throughout the entire pellet. Sometimes a somewhat higher load was observed on the outside of the pellet.

## Example 2

0.69 g Sn(II)oxalate (Fluka A.G., purum) was dissolved in a solution of 60% by weight formic acid (Merck, 'reinst'). The pH was increased with concentrated ammonia (25% by weight) to a value of 3.9. A solution with a volume of 25.6 ml resulted. This solution contains the bivalent tin as the Sn-

$(HCO_2)_3$ complex. The formate ion, which is a stronger ligand than the hydroxide ion, prevents hydrolysis of the tin(II).

78.84 g alpha-aluminum oxide pellets (Norton SA 5525 HPC) were impregnated with the above solution by the incipient wetness procedure. These pellets are cylindrical with a diameter of about 5 mm and a length of about 7 mm and have a BET area of 0.27 $m^2$/g.

The pellets thus prepared were dried for about 4 hours at 120°C, and on the basis of Sn(O) contain 0.50% by weight of Sn. Subsequently, the BET area was measured after 24 hours' calcination as a function of the calcination temperature. After drying at 120°C, no increase in area was to be measured relative to that of the pure carrier material. This indicates that there has been no decomposition of the complex and that, accordingly, no $SnO_2$ particles have as yet formed. After calcination at 300°C, an area of 1.82, +/- 0.06 $m^2$/g was measured; at 500°C 1.31, +/- 3.31 $m^2$/g; at 750°C 0.88 $m^2$/g; and at 1250°C no increase in area was observed relative to the pure carrier material.

The above results indicate that the $SnO_2$ is sintered as a function of the temperature after first being deposited on the $Al_2O_3$ surface as extremely small particles at low temperature. This was confirmed by mercury porosimetry. Electron microscopy confirmed the above picture, and in addition it was shown by means of elemental analysis in the scanning microscope that the $SnO_2$ distribution is homogeneous throughout the entire pellet. Sometime, a somewhat higher load was observed on the outside of the pellet.

## Example 3

In this example, tablets of alpha $Al_2O_3$ were loaded with tin oxide. The tablets (Norton HPC 5525) were cylindrical rings with a diameter of about 5 mm and about 7 mm long. The B.E.T. area of the material was 0.27 $m^2$ Mercury penetration showed that this carrier material mainly contained macropores. The penetrated quantity of mercury showed the pore volume was 0.40 ml/g.

During the so-called wet impregnation, 0.40 to 0.45 ml solution per g carrier was added. Accordingly, this is slightly more than required to fully fill the pore volume.

For the impregnation tin citrate was used. The solution was obtained by suspending tin (II) oxalate and citrate in equimolar ratio in de-ionized water. Subsequently, the pH value was carefully adjusted to a value of between 5.5 and 6.0 by slowly adding concentrated ammonia. The concentration of the solution was adjusted so that, after impregnation with 0.40 to 0.45 ml/g a degree of loading of 0.96 % by weight is obtained.

After impregnation, several samples of the material were rapidly calcined at various temperatures ranging from 350 to 1250° C. An examination in the electron microscope showed that the carrier material was coated with a uniform layer of tin oxide. It turned out that calcination at temperatures of about 400° C breaks up the uniform layer into discrete tin oxide particles. Particle size was determined from broadening of the X-ray refractions. It increased from 9.5 nm after calcination at 500° C to 128.0 nm after calcination at 1250° C.

## Claims

1. A process for preparing a carrier material for loading with a catalytically active material or with a precursor of a catalytically active material, characterized by impregnating the carrier which has a surface area of less than 20 $m^2/g$ with a solution of a complex of tin or iron and citric acid, formic acid, lactic acid or oxalic acid in a solvent, the viscosity of which solution is not decreased upon heating and/or evaporation of the solvent, subsequently evaporating the solvent and decomposing the complex by heating.

2. A process as claimed in claim 1, characterized by using a solution of a complex of the active component, whose viscosity is increased upon heating and evaporation of the solvent.

3. A process as claimed in claim 1 or 2, characterized by using a solution of a complex of the active component in which the crystallization of the solid complex does not proceed, or very slowly, upon heating and evaporation of the solvent.

4. A process as claimed in claims 1-3, characterized by impregnating the bodies of the carrier material with so much of the solution of the complex as is needed just to fill the pore volume of the carrier.

5. A process as claimed in claims 1-4, characterized in that, prior to the impregnation with the solution of the complex, the carrier material is evacuated, optionally at elevated temperature.

6. A process as claimed in claims 1-5, characterized by adding to the said solution sugar, glucose, fructose, or a similar compound that is poorly crystallizable and readily soluble.

7. A process as claimed in claims 1-6, characterized in that any carbonaceous products remaining after decomposition of the complex are removed by oxidation at elevated temperature.

8. A process according to claim 1-7, characterized in that the surface area of the carrier is less than 15 $m^2/g$, more in particular less than 10 $m^2/g$.

9. A process as claimed in any one of the preceding claims, characterized by applying a finely-divided, thermostable material capable of functioning as a carrier material to a non-porous structure, such as, for example, a honeycomb.

10. Shaped products produced by the process as claimed in any of claims 1-9.

## Revendications

1. Procédé de préparation d'un matériau support destiné à être chargé avec un matériau catalytiquement actif ou avec un précurseur d'un matériau catalytiquement actif, caractérisé en ce que l'on imprègne le support, qui présente une aire spécifique inférieure à 20 $m^2/g$, avec une solution, dans un solvant, d'un complexe de l'étain ou du fer et de l'acide citrique, de l'acide formique, de l'acide lactique ou de l'acide oxalique, la viscosité de cette solution ne diminuant pas à la suite d'un chauffage et/ou d'une évaporation du solvant, et en ce qu'ensuite, on évapore le solvant et on décompose le complexe par chauffage.

2. Procédé conforme à la revendication 1, caractérisé en ce que l'on utilise une solution d'un complexe du composant actif, solution dont la viscosité augmente à la suite d'un chauffage et d'une évaporation du solvant.

3. Procédé conforme à la revendication 1 ou 2, caractérisé en ce qu'on utilise une solution d'un complexe du composant actif, dans laquelle la cristallisation du complexe solide ne se produit pas, ou ne se produit que très lentement, à la suite d'un chauffage et d'une évaporation du solvant.

4. Procédé conforme aux revendications 1 à 3, caractérisé en ce qu'on imprègne les morceaux du matériau support avec la quantité de solution de complexe juste nécessaire pour remplir le volume des pores du support.

5. Procédé conforme aux revendications 1 à 4, caractérisé en ce que, avant l'imprégnation avec la solution de complexe, le matériau support est purgé, éventuellement à température élevée.

6. Procédé conforme aux revendications 1 à 5, caractérisé en ce que l'on ajoute à ladite solution du sucre, du glucose, du fructose ou un composé semblable qui est faiblement cristallisable et facilement soluble.

7. Procédé conforme aux revendications 1 à 6, caractérisé en ce que tout produit carboné restant après la décomposition du complexe est éliminé par oxydation à température élevée.

8. Procédé conforme aux revendications 1 à 7, caractérisé en ce que l'aire spécifique du support vaut moins de 15 m²/g, plus particulièrement moins de 10 m²/g.

9. Procédé conforme à l'une quelconque des revendications précédentes, caractérisé en ce qu'on applique un matériau stable à la chaleur, finement divisé et capable de jouer le rôle de matériau support, sur une structure non poreuse, comme par exemple une structure en nid d'abeilles.

10. Produit façonné, fabriqué selon un procédé conforme à l'une quelconque des revendications 1 à 9.

**Patentansprüche**

1. Verfahren zur Herstellung eines Trägermaterials für das Beladen mit einem katalytisch aktiven Material oder mit einem Vorläufer eines katalytisch aktiven Materials, dadurch gekennzeichnet, daß man den Träger, der eine Oberfläche von weniger als 20 m²/g hat, mit einer Losung eines Komplexes von Zinn oder Eisen und Zitronensäure, Ameisensäure, Milchsäure oder Oxalsäure in einem Lösungsmittel imprägniert, wobei die Viskosiät dieser Lösung nicht bei Erhitzen und/oder Abdampfen des Lösungsmittels abnimmt, und daß man anschließend das Lösungsmittel abdampft und den Komplex durch Erhitzen zersetzt.

2. Verfahren nach Anspruch 1, dadurch gekennzeichnet, daß man eine Lösung eines Komplexes des aktiven Bestandteils verwendet, deren Viskosität beim Erhitzen und Abdampfen des Lösungsmittels erhöht wird.

3. Verfahren nach Anspruch 1 oder 2, dadurch gekennzeichnet, daß man eine Lösung eines Komplexes des aktiven Bestandteils verwendet, worin die Kristallisation des festen Komplexes bei Erhitzen und Abdampfen des Lösungsmittels nicht oder sehr langsam fortschreitet.

4. Verfahren nach den Ansprüchen 1-3, dadurch gekennzeichnet, daß man die Körper des Trägermaterials mit so viel der Lösung des Komplexes imprägniert, wie gerade zum Füllen des Porenvolumens des Trägers erforderlich ist.

5. Verfahren nach den Ansprüchen 1-4, dadurch gekennzeichnet, daß vor der Imprägnierung mit der Lösung des Komplexes das Trägermaterial evakuiert wird, gegebenenfalls bei einer erhöhten Temperatur.

6. Verfahren nach den Ansprüchen 1-5, dadurch gekennzeichnet, daß man zur Lösung Zucker, Glucose, Fructose oder eine ähnliche Verbindung zugibt, die schlecht kristallisierbar und leicht löslich ist.

7. Verfahren nach den Ansprüchen 1-6, dadurch gekennzeichnet, daß kohlenstoffhaltige Produkte, die nach Zersetzung des Komplexes zurückbleiben, durch Oxidation bei erhöhter Temperatur entfernt werden.

8. Verfahren nach den Ansprüchen 1-7, dadurch gekennzeichnet, daß die Oberfläche des Trägers weniger als 15 m²/g, insbesondere weniger als 10 m²/g ist.

9. Verfahren nach einem der vorhergehenden Ansprüche, dadurch gekennzeichnet, daß man ein fein verteiltes, hitzestabiles Material aufbringt, das als Trägermaterial auf einer nicht porösen Struktur wie etwa z.B. einer Wabe dienen kann.

10. Geformte Produkte, hergestellt durch ein Verfahren nach einem der Ansprüche 1-9.